**Europäisches Patentamt**

**European Patent Office**

**Office européen des brevets**

(19)

(11) Veröffentlichungsnummer : **0 347 572 B1**

(12)

# EUROPÄISCHE PATENTSCHRIFT

(45) Veröffentlichungstag der Patentschrift :
**15.07.92 Patentblatt 92/29**

(51) Int. Cl.$^5$ : **A61K 6/04**

(21) Anmeldenummer : **89108462.6**

(22) Anmeldetag : **11.05.89**

(54) **Zahnfüllmaterial und Verfahren zu dessen Herstellung.**

(30) Priorität : **22.06.88 DE 3820970**

(43) Veröffentlichungstag der Anmeldung :
**27.12.89 Patentblatt 89/52**

(45) Bekanntmachung des Hinweises auf die
Patenterteilung :
**15.07.92 Patentblatt 92/29**

(84) Benannte Vertragsstaaten :
**AT BE CH DE ES FR GB GR IT LI LU NL SE**

(56) Entgegenhaltungen :
**DE-C- 3 403 779**

(73) Patentinhaber : **Degussa Aktiengesellschaft
Weissfrauenstrasse 9
W-6000 Frankfurt am Main 1 (DE)**

(72) Erfinder : **Diehl, Walter, Dr. Dipl.-Phys.
Gustav-Adolf-Strasse 29
W-6450 Hanau 1 (DE)**
Erfinder : **Ringelstein, Hans-Martin
Inheidener Strasse 22
W-6000 Frankfurt 60 (DE)**

EP 0 347 572 B1

## Beschreibung

Die Erfindung betrifft ein Zahnfüllmaterial aus einem porösen Goldsinterkörper zum Einbringen in Kavitäten und Verfestigen unter Einwirkung von Ultraschall.

In der konservierenden Zahnheilkunde sind eine Reihe von metallischen Füllstoffen bekannt, wie beispielsweise Amalgame, Gußlegierungen in Form in Inlays oder Stopfgold. Die Füllung der Zahnkavitäten mit Stopfgold ist eine der ältesten Zahnfüllungsmethoden. Für diese Goldstopffüllungen wird chemisch reines Gold in Form von Goldfolie, Goldschwamm oder Goldpulver verwendet.

Die aus reinem Gold hergestellten Goldstopffüllungen werden in Bezug auf Haltbarkeit, Aestethik und Korrosionsbeständigkeit ausgezeichnet beurteilt. Schwerwiegende Nachteile der Goldstopffüllungen sind jedoch das technisch und zeitlich sehr aufwendige Präparieren der Kavität und das ebenfalls großes Geschick erfordernde Legen der Füllung. So ist zunächst eine sehr sorgfältige Bearbeitung der Kavität mit Unterschnitten und eine nicht automatisch durchführbare Aufrauhung der Kavitätenwände nötig. Beides ist unbedingt Voraussetzung für eine ausreichende Haftung des Goldes in der Kavität. Desweiteren muß die Kavität während des Goldstopfvorganges absolut frei von Feuchtigkeit sein. Dies betrifft nicht nur den Speichelfluß, sondern auch die Atemluft des Patienten.

Dies macht die ebenfalls zeitraubende und für den Patienten mitunter sehr unangenehmen Anwendung von sogenannten Cofferdam-Folien erforderlich. Darüberhinaus muß das Material der Goldstopffüllung unmittelbar vor dem Einbringen in die Kavität in einer sehr sauberen Alkoholflamme ausgeglüht werden, damit sämtliche Verunreinigungen auf der Oberfläche entfernt werden und eine kohäsive Bindung zwischen den einzelnen Goldteilchen erreicht wird. Die Kaltschweissbarkeit des Goldes, die Grundlage des Goldstopfens, wird durch eine Kontamination der Oberfläche, insbesondere durch Flüssigkeitsfilme, sehr stark vermindert.

Aus der DE-PS 30 42 008 ist ein Goldstopfverfahren bekannt, bei dem ein poröser Sinterkörper oder ein Drahtgeflechtknäuel, vorzugsweise aus Gold, zusammen mit einem im Temperaturbereich von 15 bis 40°C sich verfestigenden, plastischen oder flüssigen organischen Bindemittel, wie Methylmethacrylat oder sonstige in der Zahntechnik übliche Kunststoffe oder Zahnzemente, in die Kavität eingebracht und mit manuellen Stopfgeräten der Kavität angepasst und verfestigt wird. Die Goldsinterkörper besitzen hierbei ein Porenvolumen von 58 bis 66 %. Mit diesem Verfahren lassen sich allerdings keine einwandfreien Oberflächen erzeugen, die außerdem nicht rein metallisch sind, sondern noch den organischen Binder, meist Methacrylate, enthalten.

Aus der US-PS 1,040,972 ist ein plastisches Goldmaterial bekannt, bestehend aus Goldfasern oder Goldschwamm, getränkt mit einem in der Wärme weichen, beim Abkühlen hartwerdenden Wachs, das auch zur Herstellung von Zahnfüllungen verwendet werden kann. Auch hier lassen sich keine einwandfreien Oberflächen erzeugen, da das Wachs in der Zahnfüllung verbleibt.

Aus der DE-PS 3403779 ist ein Zahnfüllmaterial zum Einbringen in Kavitäten und Verfestigung unter Einwirkung von Ultraschall bekannt, das aus plättchenförmigen Goldpulver und Polyäthylenglycol in Mengen von 0,5 bis 5 Gew.-% besteht. Nachteilig ist auch hier die Anwesenheit eines organischen Bindemittels in der Zahnfüllung.

Es war daher Aufgabe der vorliegenden Erfindung ein Zahnfüllmaterial aus einem porösen Goldsinterkörper zum Einbringen in Kavitäten und Verfestigen unter Einwirkung von Ultraschall zu entwickeln, das ohne die Verwendung von organischen Bindemitteln auskommt.

Diese Aufgabe wird erfindungsgemäß dadurch gelöst, daß der Sinterkörper aus plättchenförmigen durch chemische Fällung hergelltem Goldpulver besteht und ein Porenvolumen von 80 bis 90 % aufweist.

Vorzugsweise besitzt der Sinterkörper Streifenform.

Die Herstellung dieses Zahnfüllmaterials erfolgt vorteilhafterweise so, daß plättchenförmiges, durch chemische Fällung erzeugtes Goldpulver ohne verdichtendes Vorpressen bei 300 bis 900°C zu einem porösen Formkörper mit einem Porenvolumen von 80 bis 90 % gesintert wird.

Vorzugsweise besitzen die Goldplättchen eine Größe von 5 bis 100µm, bei einer Dicke von 0,1 bis 5 µm.

Weiterhin ist es günstig, wenn das Sintern der Goldplättchen während 15 bis 45 Minuten bei 500 bis 700°C durchgeführt wird, insbesondere bei 550 bis 640°C.

Überraschenderweise lassen sich mit diesen Sinterkörpern unter Einwirkung von Ultraschall Goldstopffüllungen herstellen, die eine gute Haltbarkeit aufweisen und eine rein metallische Oberfläche besitzen. Außerdem besitzen diese Sinterkörper eine solche mechanische Konsistenz, die ein problemloses Nachführen und Abscheren des Materials in der Kavität erlauben.

Die Herstellung dieser Sinterkörper erfolgt auf die Weise, daß ein nach einem bekannten Verfahren durch chemische Fällung hergestelltes plättchenförmiges Goldpulver in loser Schüttung ohne Vorverdichtung in einer geeigneten Form bei Temperaturen von 300 bis 900°C gesintert wird, wobei ein poröser Goldformkörper entsteht, der ein Porenvolumen von 80 bis 90 % aufweisen muß. Bei Verwendung von mechanisch hergestellten Goldplättchen ist der Sinterkörper zum Füllen von Zahnkavitäten nicht geeignet.

Folgendes Beispiel soll die Erfindung näher erläutern:

Plättchenförmiges Goldpulver einer mittleren Teilchengröße von 15x0,3 $\mu m^2$ wurde aus einer salzsauren Goldsalzlösung in Anwesenheit von Schutzkolloiden durch Reduktion mit einem ungesättigten Alkohol ausgefällt. Dieses Goldpulver wurde in eine oben offene Rinne mit rechteckigem Querschnitt (30x1,5x0,3 $mm^3$) eingestreut und 30 Minuten bei 600°C an der Luft gesintert. Der so hergestellte Streifen besaß ein Porenvolumen von 83 % und wurde nach und nach in eine Zahnkavität eingeführt, wo das Material mit einem Ultraschallwerkzeug (f=40 KHz) mit einer Anpreßkraft von 3 bis 5 N verdichtet und verfestigt wurde. Die fertige Zahnfüllung besaß eine Scherfestigkeit von 60 N·mm$^{-2}$.

## Patentansprüche

1. Zahnfüllmaterial aus einem porösen Goldsinterkörper zum Einbringen in Kavitäten und Verfestigen unter Einwirkung von Ultraschall, dadurch gekennzeichnet, daß der Sinterkörper aus plättchenförmigem, durch chemische Fällung hergestelltem Goldpulver besteht und ein Porenvolumen von 80 bis 90 % aufweist.

2. Zahnfüllmaterial nach Anspruch 1, dadurch gekennzeichnet, daß der Sinterkörper Streifenform aufweist.

3. Verfahren zur Herstellung von Zahnfüllmaterial nach Anspruch 1 und 2, dadurch gekennzeichnet, daß plättchenförmiges, durch chemische Fällung erzeugtes Goldpulver ohne verdichtendes Vorpressen bei 300 bis 900°C zu einem porösen Formkörper mit einem Porenvolumen von 80 bis 90 % gesintert wird.

4. Verfahren nach Anspruch 3, dadurch gekennzeichnet, daß die Goldplättchen eine Größe von 5 bis 100 $\mu m$, bei einer Dicke von 0,1 bis 5 $\mu m$ besitzen.

5. Verfahren nach Anspruch 3 und 4, dadurch gekennzeichnet, daß das Sintern der Goldplättchen während 15 bis 45 Minuten bei 500 bis 700°C durchgeführt wird.

## Claims

1. A dental filling material of a porous gold sintered compact for introduction into cavities and hardening under the effect of ultrasound, characterized in that the sintered compact consists of platelet-like gold powder produced by chemical precipitation and has a pore volume of 80 to 90%.

2. A dental filling material as claimed in claim 1, characterized in that the sintered compact is in the form of a strip.

3. A process for the production of the dental filling material claimed in claims 1 and 2, characterized in that platelet-like gold powder produced by chemical precipitation is sintered without preliminary compaction at 300 to 9300°C to form a porous moulding having a pore volume of 80 to 90%.

4. A process as claimed in claim 3, characterized in that the gold platelets are between 5 and 100 $\mu m$ in size for a thickness of 0.1 to 5 $\mu m$.

5. A process as claimed in claims 3 and 4, characterized in that the gold platelets are sintered for 15 to 45 minutes at 500 to 700°C.

## Revendications

1. Matériau de remplissage de dents constitué d'un fritté en or, destiné à être introduit dans des cavités et à être durci par ultrason, caractérisé en ce que le corps fritté est composé de poudre d'or lamellaire, produite par précipitation chimique et a un volume de pores de 80 à 90 %.

2. Matériau de remplissage de dents selon la revendication 1, caractérisé en ce que le corps fritté se présente sous forme de bande.

3. Procédé pour la préparation de matériau de remplissage de dents selon les revendications 1 et 2, caractérisé en ce que la poudre d'or lamellaire, obtenue par précipitation chimique sans compression est frittée à une température de 300 à 900°C en un corps de forme poreux ayant un volume de pores de 80 à 90 %.

4. Procédé selon la revendication 3, caractérisé en ce que les lamelles d'or ont une grandeur de 5 à 100 x 10$^{-6}$m et une épaisseur de 0,1 à 5 x 10$^{-6}$m.

5. Procédé selon les revendications 3 et 4, caractérisé en ce que le frittage des lamelles d'or est effectué durant 15 à 45 minutes et à une température de 500 à 700°C.